# EUROPEAN PATENT APPLICATION

(11) **EP 4 186 445 A1**
(43) Date of publication of application: **31.05.2023**
(21) Application number: 21818862.1
(22) Date of filing: 23.07.2021
(51) Int. Cl.: A61B 17/12

(54) **RELEASE APPARATUS, RELEASE SYSTEM, RELEASE METHOD, AND TREATMENT APPARATUS**

(71) Applicant: MicroPort NeuroTech (Shanghai) Co., Ltd., Shanghai 201318 (CN)
(72) Inventor: WANG, Xingzhao, Shanghai 201318 (CN); JIANG, Tao, Shanghai 201318 (CN); WEI, Lin, Shanghai 201318 (CN); WANG, Yiqun Bruce, Shanghai 201318 (CN)
(74) Representative: Marks & Clerk LLP
(86) International application number: PCT/CN2021/108015
(87) International publication number: WO 2021/244670

(57) **Abstract**

A release apparatus (100), a release system, a release method, and a treatment apparatus are disclosed. The release apparatus (100) uses two indicators, electric current and power comsuption, to determine whether the release process performed by the release system is successful. This reduces misjudgments, improves the accuracy of the determination of the release state. The release system also uses a self-loop structure, such that it is unnecessary to insert a conductive needle or electrode into the body of a patient, reducing the suffering of the patient.

## Description

### TECHNICAL FIELD

The present invention relates to the technical field of medical instruments and, in particular, to a release apparatus, a release system, a release method, and a treatment apparatus.

### BACKGROUND

Intracranial aneurysm is a common vascular disease that threatens middle-aged and elderly people. The aneurysm ruptures in patients due to high blood pressure. If no timely and effective treatment is given, it will be life-threatening. The traditional treatment method for intracranial aneurysm is mainly to clip the aneurysm by surgical operation, but it has the disadvantages of large trauma and high patient mortality.

In recent years, with the rapid development of interventional therapy technology, more and more patients with intracranial aneurysm choose interventional therapy. A commonly used interventional treatment method for intracranial aneurysm is to use a coil for embolization. The coil is connected to a push rod. The doctor pushes the push rod to deliver the coil to the diseased site where the intracranial aneurysm is located, and pushes the coil into the lumen of the aneurysm. After the coil is in place, the coil needs to be separated from the push rod, which requires a release process to be performed. The release process is carried out in the patient's body, and the doctor cannot observe the progress of the release. Therefore, how to determine that the coil has been completely released has become an urgent problem to be solved.

### SUMMARY OF THE INVENTION

An object of the present invention is to provide a release apparatus, a relase system, a release method, and a treatment apparatus, which can reduce the probability of misjudgment of the release state and improve the judgment accuracy of the release state.

In order to achieve the above object, the present invention provides a release system, comprising a release apparatus and a push rod; wherein,
the release apparatus comprises a power supply assembly, a current detector and a control unit;
the power supply assembly is configured to connect with the push rod, and supply power to positive and negative electrodes of the push rod, and the push rod is configured to connect with an implant;
when the power supply assembly supplies power to the positive and negative electrodes of the push rod, the release system forms a release circuit;
the current detector is configured to detect a current of the release circuit;
the control unit is in communication with the current detector to acquire a power consumption of the release circuit from the current detector, and configured to determine whether a release process performed by the release system is successful according to the power consumption and the current of the release circuit.

Optionally, the control unit pre-stores a current threshold and a power consumption threshold;
when determining whether the release process performed by the release system is successful, the control unit is configured to determine whether the present current of the release circuit is less than the current threshold, and whether the present power consumption of the release circuit is greater than the power consumption threshold; if the present current of the release circuit is less than the current threshold and the present power consumption of the release circuit is greater than the power consumption threshold, the control unit determins that the release process of the release system is successful.

Optionally, the release apparatus further comprises a timer that is in communication with the control unit and configured to detect a power-on duration of the release circuit;
the control unit is configured to calculate the power consumption of the release circuit according to the power-on duration and the current of the release circuit.

Optionally, the control unit further pre-stores a release period;
when the present current of the release circuit is greater than or equal to the current threshold and the persent power consumption is greater than the power consumption threshold, or when the present current of the release circuit is less than the current threshold and the present power consumption is less than or equal to the power consumption threshold, the control unit is further configured to determine whether the power-on duration is less than the release period; if not, the control unit determines that the release process fails, and de-energizes the release circuit to cease the release process; and if so, the control unit determines to continue the release process.

Optionally, the power consumption of the release circuit is an integral of the current of the release circuit with respect to the power-on duration.

Optionally, the push rod comprises a first electrical conductor and a second electrical conductor, the first electrical conductor configured to connect with the negative electrode of the power supply assembly, the second electrical conductor comprising a conductive area and a release area connnected with each other, the conductive area configured to connect with the positive electrode of the power supply assembly, and the release area configured to connect with the first electrical conductor.

Optionally, the power supply assembly comprises a constant current source and a current controller, and wherein both the constant current source and the current controller are in communication with the control unit, and the constant current source is connected with the push rod by the current controller.

Optionally, the release apparatus further comprises a display element that is in communication with the control unit and configured to display a release state.

In order to achieve the above object, the present invention also provides a treatment apparatus, comprising an implant and the above release system, wherein the implant is configured to connect with the push rod, and can be disconnected from the push rod under a predetermined condition.

Optionally, the push rod comprises a release area, to which the implant is configured to connect, and wherein the release area is broken under the predetermined condition to disconnect the implant from the push rod.

Optionally, the predetermined condition is that, when the release system forms the release circuit, a present current of the release circuit is less than a predetermined current threshold, and a power consumption of the release circuit is greater than a predetermined power consumption threshold.

In order to achieve the above object, the present invention also provides a release apparatus, comprising a power supply assembly, a control unit and a current detector; wherein,
the power supply assembly is configured to connect with a push rod, and supply power to positive and negative electrodes of the push rod to form a release circuit;
the current detector is configured to detect a current of the release circuit; and
the control unit is in communication with the current detector to acquire a power consumption of the release circuit, and configured to determine whether a release process performed by the release system is successful according to the power consumption and the current of the release circuit.

Optionally, the control unit pre-stores a current threshold and a power consumption threshold;
when determining whether the release process of the release system is successful, the control unit is configured to determine whether the present current of the release circuit is less than the current threshold, and whether the present power consumption of the release circuit is greater than the power consumption threshold; if the present current of the release circuit is less than the current threshold and the present power consumption of the release circuit is greater than the power consumption threshold, the control unit determins that the release process of the release system is successful.

Optionally, the release apparatus further comprises a timer that is in communication with the control unit and configured to detect a power-on duration of the release circuit;
the control unit is configured to calculate the power consumption of the release circuit according to the power-on duration and the current of the release circuit.

Optionally, the control unit further pre-stores a release period;
when the present current of the release circuit is greater than or equal to the current threshold and the persent power consumption is greater than the power consumption threshold, or when the present current of the release circuit is less than the current threshold and the present power consumption is less than or equal to the power consumption threshold, the control unit is further configured to determine whether the power-on duration is less than the release period; if not, the control unit determines that the release process fails, and de-energizes the release circuit to cease the release process; and if so, the control unit determines to continue the release process.

Optionally, the power consumption of the release circuit is an integral of the current of the release circuit with respect to the power-on duration.

Optionally, the power supply assembly comprises a constant current source and a current controller connected with each other, and wherein both the constant current source and the current controller are in communication with the control unit.

Optionally, the release apparatus further comprises a display element that is in communication with the control unit and configured to display a release state.

In order to achieve the above object, the present invention also provides a release method, applied to a release circuit of a release system, wherein the release method comprises:
Step S 1: detecting a present current of the release circuit;
Step S2: acquiring a power consumption of the release circuit;
Step S3: determining whether a release process performed by the release system is successful according to the present current and the present power consumption of the release circuit.

Optionally, acquiring the power consumption of the release circuit is achieved by:
acquiring a power-on duration of the release circuit;
calculating the power consumption of the release circuit according to the current and the power-on duration of the release circuit.

Optionally, Step S3 is performed as follows:
Step S31: determining whether the present current of the release circuit is less than a current threshold, and whether the present power consumption of the release circuit is greater than a power consumption threshold; if the presnet current of the release circuit is less than the current threshold and the present power consumption of the release circuit is greater than the power consumption threshold, it is determined that the release process is successful; if the present current of the release circuit is greater than or equal to the current threshold and the present power consumption of the release circuit is greater than the power consumption threshold, or if the present current is less than or equal to the present current threshold and the present power consumption of the release circuit is less than the power consumption threshold, it is directed to Step S32;
Step S32: determining whether the power-on duration is less than a preset release period; if not, it is determined that the release process fails, and if so, it returns to Step S1.

Compared with the prior art, the release apparatus, system and method and treatment apparatus of the present invention have the following advantages:

The aforesaid release system includes a release apparatus and a push rod. The release apparatus includes a power supply assembly, a control unit and a current detector. The power supply assembly is configured to connect with the push rod, and supply power to the positive and negative electrodes of the push rod that is configured to connect to an implant. When the power supply assembly supplies power to the positive and negative electrodes of the push rod, the release system forms a release circuit. The current detector is configured to detect the current of the release circuit. The control unit is in communication with the current detector to acquire the power consumption of the release circuit, and determine whether the release process performed by the release system is successful according to the power consunption and the current of the release circuit. The current and the power consumption of the release circuit are taken together as the indicators for determining whether the release process is successful or not, so as to reduce the probability of misjudgment and improve the accuracy of judgment as much as possible. Moreover, the push rod can be electrically connected with the release apparatus to form a release circuit, so that the release system has a self-loop structure. As a result, there is no need to place conductive needles or electrodes on the patient's body during use, thereby reducing the suffering of the patient.

### BRIEF DESCRIPTION OF THE DRAWINGS

Fig. 1 is a schematic structural diagram of a treatment apparatus according to an embodiment of the present invention;
Fig. 2 is an overall frame diagram of a release apparatus according to an embodiment of the present invention;
Fig. 3 is a flow chart showing the release process of a release system according to an embodiment of the present invention.

### [List of Reference Numerals]:

100: release apparatus;
110: power supply assembly;
111: constant current source, 112: current controller;
120: control unit;
121: storage module;
130: current detector;
140: timer;
150: display element;
200: push rod;
210: first electrical conductor;
220: second electrical conductor;
221: conductive area, 222: release area;
230: insulation;
300: implant;
S: release circuit.

### DETAILED DESCRIPTION

Objectives, advantages and features of the present invention will become more apparent upon reading the following more detailed description of the present invention, which is set forth by way of particular embodiments with reference to the accompanying drawings. Note that the figures are provided in a very simplified form not necessarily drawn to exact scale and for the only purpose of facilitating easy and clear despription of the embodiments.

As used herein, the singular forms "a", "an" and "the" include plural referents, unless the context clearly dictates otherwise. As used herein and in the appended claims, the term "or" is generally employed in the sense of "and/or", "plurality" of "two or more", and "several" of "one or more", unless the context clearly dictates otherwise. As used herein, unless otherwise expressly specified and defined, the terms "mounting", "coupling" and "connection" should be interpreted in a broad sense. For example, a connection may be a fixed, detachable or integral connection, or a mechanical or electrical connection, or a direct or indirect connection with one or more intervening media, or an internal communication or interaction between two components. Those of ordinary skill in the art can understand the specific meanings of the above-mentioned terms herein, depending on their context. The same or similar reference numerals in the drawings represent the same or similar parts.

As used herein, a "proximal end" and a "distal end" are relative orientations, relative positions and directions of elements or actions relative to each other from the perspective of the user operating the medical instruments. Although the "proximal end" and the "distal end" are not restrictive, the "proximal end" generally refers to the end of the medical device that is close to the user during normal operation, while the "distal end" generally refers to the end that enters the patient's body first.

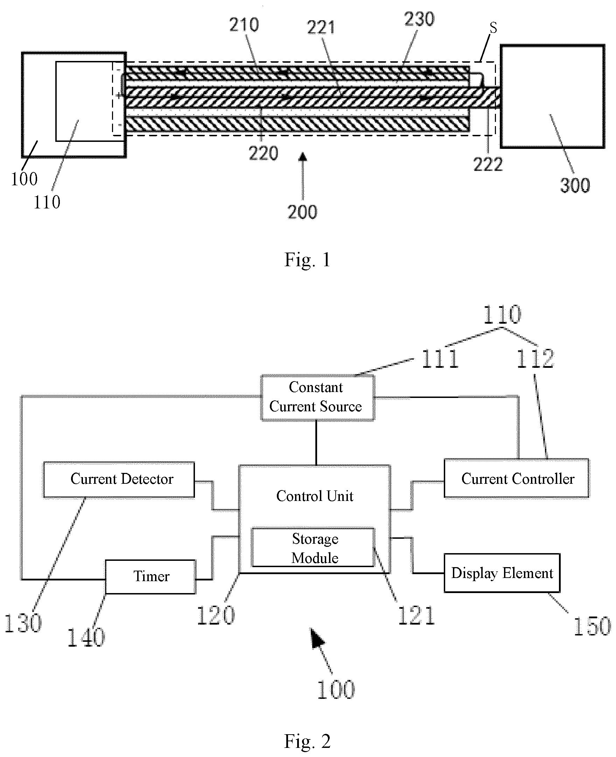

The present invention provides a release apparatus that is applied to a release system. The release system is configured to deliver an implant to a predetermined position in a patient's body. The implant includes but is not limited to an embolic coil. As shown in Fig. 1, the release system includes a release apparatus 100 and a push rod 200. The push rod 200 includes a release area 222 having a distal end connected with an implant 300, and a proximal end provided with positive and negative electrodes both configtured for electrical connection with the release apparatus 100. In actual use, the operator operates the proximal end of the push rod 200 to deliver the implant 300 to a predetermined position in the patient's body. When the surgical operation is completed and the implant 300 needs to be released, the proximal end of the push rod 200 is connected to the release apparatus 100, and the release apparatus 100 supplies power to the positive and negative electrodes of the push rod 200 to form a release circuit S so that the release area 222 of the push rod 200 can be broken to complete the release of the implant 300. The principle of supplying power to the positive and negative electrodes of the push rod 200 to ionize and break the release area 222 is a common knowledge in the art, and will not be described here.

Fig. 2 is an overall frame diagram of a release apparatus 100 according to an embodiment of the present invention. Please refer to Fig. 2 and in conjunction with Fig. 1, the release apparatus 100 includes a power supply assembly 110, a control unit 120, and a current detector 130. The power supply assembly 110 is configured to connect with the push rod 200 and supply power to the positive and negative electrodes of the push rod 200 to form a release circuit S. The current detector 130 is in communication with the control unit 120, and configured to detect the current of the release circuit S and send the information about the current to the control unit 120. The control unit 120 is configured to acquire the power consumption of the release circuit S, and determine whether the release process performed by the release system is successful according to the current and the power consumption.

Further, the control unit 120 includes a storage module 121 pre-storing a current threshold and a power consumption threshold. In the process of determining whether the release process of the release system is successful, the control unit 120 is configured to determine whether the present current of the release circuit S is less than the current threshold, and whether the present power consumption of the release circuit S is greater than the power consumption threshold, if the current of the release circuit S is less than the current threshold, and the power consumption of the release circuit S is greater than the power consumption threshold, it is determined that the release process is successful.

In the release process, the current of the release circuit S changes in value in real time. Therefore, the release state of the release system can be determined by detecting the value of the current. If the release area 222 has been broken, the resistance in the release circuit S is generally very large, and the current of the release circuit S will become very small at this time. The control unit 120 can thus determine whether the release process is successful according to the change of the current of the release circuit S. However, malfunction may occur in other elements of the release circuit S, which also increases the resistance of the release circuit S. In this case, the current of the release circuit S is also very small. Therefore, the release apparatus described in this embodiment is further provided with a second judgment indicator, that is, the present power consumption of the release circuit S. To determine the resease state according to the current combined with the power consumption can reduce misjudgment and improve judgment accuracy.

During the release process of the release apparatus 100, it may happen that the current and the power consumption cannot meet the judgment criteria at the same time. For example, the present current of the release circuit S is less than the current threshold, and the present power consumption of the release circuit S is less than the power consumption threshold. For another example, the present current of the release circuit S is greater than or equal to the current threshold, and the present power consumption of the release circuit S is greater than or equal to the power consumption threshold. In this regard, the release apparatus 100 is provided with a further judgment criteria.

Specifically, the release apparatus further includes a timer 140 that is in communication with the control unit 120 and configured to detect the power-on duration of the release circuit S. A release period is also pre-stored in the storge module 121. When the present current of the release circuit S is less than the current threshold and the power consumption of the release circuit S is less than the power consumption threshold, or when the present current of the release circuit S is greater than or equal to the current threshold and the present power consumption is greater than or equal to the power consumption threshold, the control unit 120 is further configured to determine whether the power-on duration is less than the release period, and if not, determine that release process fails, and de-energizes the release circuit S to cease the release process, and if so, it is determined to continue the release process.

Optionally, the control unit 120 may calculate the power consumption of the release circuit S according to the current of the release circuit S and the power-on duration. For example, the power consumption may be the integral of the current of the release circuit S with respect to the power-on duration, which can also be understood as the product of the current of the release circuit S times the power-on duration.

Optionally, the release apparatus further includes a display element 150 that is connected to the control unit 120 and configured to display the release state, for example, to display that the release process is successful, the release process fails, and the like. The display element 150 may be a buzzer alarm, a display screen, an LED light and other components.

Optionally, the power supply assembly 110 includes a constant current source 111 and a current controller 112 that are connected to each other. Both the constant current source 111 and the current controller 112 are connected to the control unit 120. The current controller 112 is configured to connect with the push rod 200. The constant current source 111 may be a DC constant current source configured to output current to the current controller 112. When the current controller 112 is turned on, the release circuit S is energized, and when the current controller 112 is turned off, the release circuit S is de-energized. The constant current source 111 can also output current directly to the control unit 120, the current detector 130, the timer 140 and the display element 150.

Further, referring to Fig. 1, an embodiment of the present invention further provides a release system including a push rod 200 and a release apparatus 100. The push rod 200 is configured for electrical connection with the release apparatus 100 to form a release circuit.

Specifically, the release apparatus 100 includes a power supply assembly 110, a control unit 120 and a current detector 130. The power supply assembly 110 is configured to connect to the push rod 200, and supply power to the positive and negative electrodes of the push rod 200. The push rod 200 is configured to connect to the implant 300. When the power supply assembly 110 supplies power to the positive and negative electrodes of the push rod 200, the release system forms the release circuit S. The current detector 130 is configured to detect the current of the release circuit S. The control unit 120 is in communication with the current detector 130. The control unit 120 is configured to acquire the power consumption of the release circuit S from the current detector 130, and determine whether the release process performed by the release system is successful according to the power consumption and the current of the release circuit S.

More specifically, please continue to refer to Fig. 1 in conjunction with Fig. 2, the power supply assembly 110 of the release apparatus 100 has a positive electrode and a negative electrode. The push rod 200 may include a first electrical conductor 210 and a second electrical conductor 220. The proximal end of the first electrical conductor 210 is configured to connect with the negative electrode of the power supply assembly 110. The second electrical conductor 220 includes a conductive area 221 and the release area 222 that are connected with each other. The proximal end of the conductive area 221 is confgiured to connect with the positive electrode of the power supply assembly 110. In other words, the proximal end of the first electrical conductor 210 forms the negative electrode of the push rod 200, and the proximal end of the conductive area 221 of the second electrical conductor 220 forms the positive electrode of the push rod 200. The release area 222 is electrically connected to the first electrical conductor 210 through an electrolyte such as body fluid (filled around the release area and the first electrical conductor in the body, not shown here). In this way, in this embodiment, the current of the release circuit S flows sequentially through the positive electrode of the power supply assembly 110, the conductive area 221 of the second electrical conductor 220, the release area 222 of the second electrical conductor 220, the electrolyte (e.g., body fluid), the first electrical conductor 210, the negative electrode of the power supply assembly 110. It shall be understood that the negative electrode and the positive electrode of the push rod 200 can be interchanged. In this embodiment, the power supply assembly 110 includes a constant current source 111 and a current controller 112 that are connected to each other. The constant current source 111 has the positive electrode and the negative electrode. The first electrical conductor 210 and the second electrical conductor 220 are connected respectively to the positive electrode and the negative electrode of the power supply assembly 110 by the current controller 112. The energization and de-energization of the of the release circuit S is controlled by controlling the on-off of the current controller 112.

Optionally, the first electrical conductor 210 and the second electrical conductor 220 have columnar structures, and the first electrical conductor 210 further has an inner cavity extending axially therethrough. The conductive area 221 of the second electrical conductor 220 is sleeved in the inner cavity and is connected to the positive electrode of the power supply assembly 110, and the release area 222 extends to the outside of the inner cavity, so that the current flows as shown by the arrows in Fig. 1 when the release circuit S is energized. Further, an insulation 230 is disposed between the first electrical conductor 210 and the conductive area 221.

An embodiment of the present invention further provides a treatment apparatus. Referring to Fig. 1, the treatment apparatus includes an implant 300 and the release system. The implant 300 is configured to connect to the distal end of the release area 222. After the operator operates the push rod 200 to deliver the implant 300 to a predetermined area in the patient's body, the push rod 200 is connected to the release apparatus 100, so that the power supply assembly 110 of the release apparatus 100 is configured for supplying power to other components of the release apparatus 100 and the positive and negative electrodes of the push rod 200 to form a release circuit S. Specifically, the power supply assembly 110 is connected to the push rod 200 by the current controller 112, so that the current controller 112 can control whether to output current to the positive and negative electrodes of the push rod 200. During the release process, the release apparatus 100 completes the release process according to a predetermined procedure.

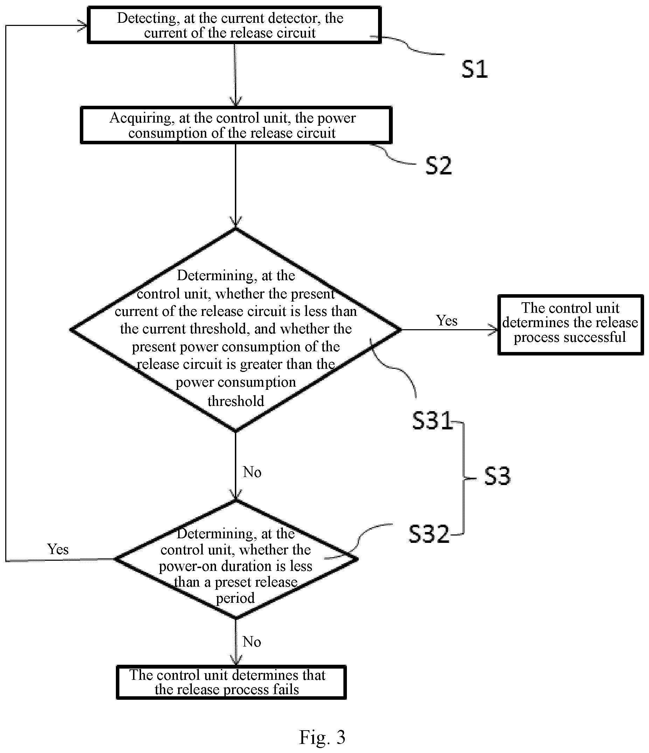

Further, the present invention also provides a release method based on a release system including a release circuit. As shown in Fig. 3, the release method comprises the following steps:
Step S 1: Detecting the current of the release circuit.
Step S2: Acquiring the power consumption of the release circuit.
Step S3: Determining whether the release process performed by the release system is successful ccording to the current and the power consumption.

The step S3 specifically includes:
Step S31: Determining whether the present current of the release circuit is less than the current threshold, and whether the present power consumption of the release circuit is greater than the power consumption threshold; if the presnet current of the release circuit is less than the current threshold and the present power consumption of the release circuit is greater than the power consumption threshold, it is determined that the release process is successful; if the present current of the release circuit is greater than or equal to the current threshold and the present power consumption of the release circuit is greater than the power consumption threshold, or if the present current is less than or equal to the present current threshold and the present power consumption of the release circuit is less than the power consumption threshold, it is directed to Step S32.

Step S32: Determining whether the power-on duration is less than a preset release period; if not, it is determined that the release process fails, and if so, it returns to Step S1.

Based on the release system provided by the embodiments of the present invention, the release method includes:
Step S1: Detecting, at the current detector, the current of the release circuit.
Step S2: Acquiring, at the control unit, the power consumption of the release circuit. Specifically, the timer acquires the power-on duration of the release circuit, and then the control unit performs integral calculation by using the current and the power-on duration, and the result thereof is the power consumption.
Step S3: Determining, at the control unit, whether the release process performed by the release system is successful according to the current and the power consumption.

The step S3 specifically includes:
Step S31: Determining, at the control unit, whether the present current of the release circuit is less than the current threshold, and whether the present power consumption of the release circuit is greater than the power consumption threshold; if the presnet current of the release circuit is less than the current threshold and the present power consumption of the release circuit is greater than the power consumption threshold, it is determined that the release process is successful; if the present current of the release circuit is greater than or equal to the current threshold and the present power consumption of the release circuit is greater than the power consumption threshold, or if the present current is less than or equal to the present current threshold and the present power consumption of the release circuit is less than the power consumption threshold, it is directed to Step S32.
Step S32: Determining, at the control unit, whether the power-on duration is less than a preset release period; if not, it is determined that the release process fails, and if so, it returns to Step S1.

In this embodiment, the current threshold, the power consumption threshold and the release period are all set by the operator according to the actual situation. For example, the current threshold may be 0.5-1.2 mA, the power consumption threshold may be 2-4.2 mAs, and the release period may be 5-20 s.

In the release apparatus, release system, release method, and treatment apparatus provided in the embodiments of the present invention, two indicators are used for the determination whether the release process is successful. This reduces misjudgment, and improves the judgment accuracy. The release system adopts a self-loop structure, so that there is no need to insert conductive needles or electrodes on the patient's body during actual use, which reduces the suffering of the patient.

Although the present invention is disclosed above, it is not limited thereto. Various modifications and variations can be made in the present invention by those skilled in the art without departing from the spirit and scope of the invention. Thus, provided that these modifications and variations of the present invention fall within the scope of the claims of the present invention and their equivalents, the present invention is also intended to include these modifications and variations.

## Claims

1. A release system, comprising a release apparatus and a push rod; wherein,
the release apparatus comprises a power supply assembly, a current detector and a control unit;
the power supply assembly is configured to connect with the push rod, and supply power to positive and negative electrodes of the push rod, and the push rod is configured to connect with an implant;
when the power supply assembly supplies power to the positive and negative electrodes of the push rod, the release system forms a release circuit;
the current detector is configured to detect a current of the release circuit;
the control unit is in communication with the current detector to acquire a power consumption of the release circuit from the current detector, and configured to determine whether a release process performed by the release system is successful according to the power consumption and the current of the release circuit.

2. The release system according to claim 1, wherein the control unit pre-stores a current threshold and a power consumption threshold;
when determining whether the release process performed by the release system is successful, the control unit is configured to determine whether the present current of the release circuit is less than the current threshold, and whether the present power consumption of the release circuit is greater than the power consumption threshold; if the present current of the release circuit is less than the current threshold and the present power consumption of the release circuit is greater than the power consumption threshold, the control unit determins that the release process of the release system is successful.

3. The release system according to claim 2, wherein the release apparatus further comprises a timer that is in communication with the control unit and configured to detect a power-on duration of the release circuit;
the control unit is configured to calculate the power consumption of the release circuit according to the power-on duration and the current of the release circuit.

4. The release system according to claim 3, wherein the control unit further pre-stores a release period;
when the present current of the release circuit is greater than or equal to the current threshold and the persent power consumption is greater than the power consumption threshold, or when the present current of the release circuit is less than the current threshold and the present power consumption is less than or equal to the power consumption threshold, the control unit is further configured to determine whether the power-on duration is less than the release period; if not, the control unit determines that the release process fails, and de-energizes the release circuit to cease the release process; and if so, the control unit determines to continue the release process.

5. The release system according to claim 3, wherein the power consumption of the release circuit is an integral of the current of the release circuit with respect to the power-on duration.

6. The release system according to claim 1, wherein the push rod comprises a first electrical conductor and a second electrical conductor, the first electrical conductor configured to connect with the negative electrode of the power supply assembly, the second electrical conductor comprising a conductive area and a release area connnected with each other, the conductive area configured to connect with the positive electrode of the power supply assembly, and the release area configured to connect with the first electrical conductor.

7. The release system according to any one of claims 1-6, wherein the power supply assembly comprises a constant current source and a current controller, and wherein both the constant current source and the current controller are in communication with the control unit, and the constant current source is connected with the push rod by the current controller.

8. The release system according to any one of claims 1-6, wherein the release apparatus further comprises a display element that is in communication with the control unit and configured to display a release state.

9. A treatment apparatus, comprising an implant and the release system according to any one of claims 1-8, wherein the implant is configured to connect with the push rod, and can be disconnected from the push rod under a predetermined condition.

10. The treatment apparatus according to claim 9, wherein the push rod comprises a release area, to which the implant is configured to connect, and wherein the release area is broken under the predetermined condition to disconnect the implant from the push rod.

11. The treatment apparatus according to claim 9 or 10, wherein the predetermined condition is that, when the release system forms the release circuit, a present current of the release circuit is less than a predetermined current threshold, and a power consumption of the release circuit is greater than a predetermined power consumption threshold.

12. A release apparatus, comprising a power supply assembly, a control unit and a current detector; wherein,
the power supply assembly is configured to connect with a push rod, and supply power to positive and negative electrodes of the push rod to form a release circuit;
the current detector is configured to detect a current of the release circuit; and
the control unit is in communication with the current detector to acquire a power consumption of the release circuit, and configured to determine whether a release process performed by the release system is successful according to the power consumption and the current of the release circuit.

13. The release apparatus according to claim 12, wherein the control unit pre-stores a current threshold and a power consumption threshold;
when determining whether the release process of the release system is successful, the control unit is configured to determine whether the present current of the release circuit is less than the current threshold, and whether the present power consumption of the release circuit is greater than the power consumption threshold; if the present current of the release circuit is less than the current threshold and the present power consumption of the release circuit is greater than the power consumption threshold, the control unit determins that the release process of the release system is successful.

14. The release apparatus according to claim 13, wherein the release apparatus further comprises a timer that is in communication with the control unit and configured to detect a power-on duration of the release circuit;
the control unit is configured to calculate the power consumption of the release circuit according to the power-on duration and the current of the release circuit.

15. The release apparatus according to claim 14, wherein the control unit further pre-stores a release period;
when the present current of the release circuit is greater than or equal to the current threshold and the persent power consumption is greater than the power consumption threshold, or when the present current of the release circuit is less than the current threshold and the present power consumption is less than or equal to the power consumption threshold, the control unit is further configured to determine whether the power-on duration is less than the release period; if not, the control unit determines that the release process fails, and de-energizes the release circuit to cease the release process; and if so, the control unit determines to continue the release process.

16. The release apparatus according to claim 14, wherein the power consumption of the release circuit is an integral of the current of the release circuit with respect to the power-on duration.

17. The release apparatus according to any one of claims 12-16, wherein the power supply assembly comprises a constant current source and a current controller connected with each other, and wherein both the constant current source and the current controller are in communication with the control unit.

18. The release apparatus according to any one of claims 12-16, wherein the release apparatus further comprises a display element that is in communication with the control unit and configured to display a release state.

19. A release method, applied to a release circuit of a release system, wherein the release method comprises:
Step S1: detecting a present current of the release circuit;
Step S2: acquiring a power consumption of the release circuit;
Step S3: determining whether a release process performed by the release system is successful according to the present current and the present power consumption of the release circuit.

20. The release method according to claim 19, wherein acquiring the power consumption of the release circuit is achieved by:
acquiring a power-on duration of the release circuit;
calculating the power consumption of the release circuit according to the current and the power-on duration of the release circuit.

21. The release method according to claim 20, wherein Step S3 is performed as follows:
Step S31: determining whether the present current of the release circuit is less than a current threshold, and whether the present power consumption of the release circuit is greater than a power consumption threshold; if the presnet current of the release circuit is less than the current threshold and the present power consumption of the release circuit is greater than the power consumption threshold, it is determined that the release process is successful; if the present current of the release circuit is greater than or equal to the current threshold and the present power consumption of the release circuit is greater than the power consumption threshold, or if the present current is less than or equal to the present current threshold and the present power consumption of the release circuit is less than the power consumption threshold, it is directed to Step S32;
Step S32: determining whether the power-on duration is less than a preset release period; if not, it is determined that the release process fails, and if so, it returns to Step S1.
